# EUROPÄISCHE PATENTSCHRIFT

(11) **EP 1 360 162 B1**
(45) Veröffentlichungstag und Bekanntmachung des Hinweises auf die Patenterteilung: **05.09.2007**
(21) Anmeldenummer: 02701274.9
(22) Anmeldetag: 13.02.2002
(51) Int. Cl.: C07C 17/156, C07C 19/045

(54) **HERSTELLUNG VON 1,2-DICHLORETHAN**
PRODUCTION OF 1, 2-DICHLOROETHANE
PRODUCTION DE 1, 2-DICHLORETHANE

(30) Priorität: 13.02.2001 DE 10107089
(43) Veröffentlichungstag der Anmeldung: 12.11.2003
(73) Patentinhaber: Vinnolit Technologie GmbH & Co. KG, 85737 Ismaning (DE)
(72) Erfinder: KAMMERHOFER, Peter, 84508 Burgkirchen (DE); MIELKE, Ingolf, 84508 Burgkirchen (DE); ERTL, Horst, 84568 Pleiskirchen (DE); STAIB, Günter, 21682 Stade (DE)
(74) Vertreter: Forstmeyer, Dietmar
(86) Internationale Anmeldenummer: PCT/EP2002/001503
(87) Internationale Veröffentlichungsnummer: WO 2002/064535

(56) Entgegenhaltungen:
- EP-A- 1 023 939
- DE-A- 4 132 030
- DE-A- 19 753 165
- DE-C- 19 837 957

## Beschreibung

Die vorliegende Erfindung bezieht sich auf ein Verfahren und eine Vorrichtung zur Herstellung von 1,2-Dichlorethan durch Umsetzen von Ethen mit Chlorwasserstoff und einem Sauerstoff aufweisenden Gas in einem Oxichlorierungsreaktor mit Wirbelbett, wobei ein Reaktionsgas gebildet wird.

Unter Oxichlorierung versteht man die Umsetzung eines Alkens - hier Ethen - mit Chlorwasserstoff und Sauerstoff beziehungsweise einem Sauerstoff enthaltenden Gas wie Luft, unter Bildung eines gesättigten chlorierten Alkans - hier 1,2-Dichlorethan, im Folgenden auch "EDC" genannt. Die Reaktion erfolgt dabei nach der Gleichung

C₂H₄ + 2HCl + ½O₂ ↔ Cl-CH₂-CH₂-Cl + H₂O.

Das Reaktionsnebenprodukt Wasser dieser Reaktion kann jedoch mit dem nicht umgesetzten Ausgangsmaterial Chlorkohlenwasserstoff die sehr stark korrosive Salzsäure bilden, so daß bei Durchführung eines solchen Verfahrens entsprechend resistente - und damit teure - Materialien für die Apparaturen zur Verfahrensdurchführung eingesetzt werden müssen.

Die Oxichlorierungsreaktion wird in Anwesenheit eines Katalysators durchgeführt.

In einer häufig im großindustriellen Ausmaß verwendeten Ausführungsform dieses Verfahrens dient als Katalysator für die Oxichlorierungsreaktion ein Katalysator-Wirbelbett, das im wesentlichen aus Kupferchlorid auf einem Aluminiumoxidträger besteht.

Bei diesen üblichen industriellen Wirbelbett-Verfahren wird der Katalysator im Oberteil des Oxichlorierungsreaktors durch mehrere hintereinander geschaltete Zyklone abgeschieden und so zum größten Teil im Reaktor zurückgehalten. Darüber hinaus geht dabei ein kleiner Anteil des Katalysators, der sogenannte Katalysatorabrieb, in das den Reaktor verlassende Reaktionsgas über und gelangt so in die 1,2-Dichlorethan-Aufarbeitung, wo er nochmals abgetrennt werden muß.

Aus der deutschen Offenlegungsschrift DE 41 32 030 ist ein Verfahren zur Entfernung des Katalysatorabriebes bekannt, der bei der Herstellung von 1,2-Dichlorethan nach dem Wirbelbett-Oxichlorierungsverfahren in der Reaktionszone anfällt und aus der Reaktionszone mit dem rohen EDC-Gasstrom herausgeführt wird. Dieses Entfernen erfolgt derart, daß man den abgeführten Katalysatorabrieb in einer trocken betriebenen Reinigungszone aus dem rohen EDC-Gasstrom abtrennt. Bevorzugte Ausführungsformen dieses Verfahrens zeichnen sich dadurch aus, daß man den Katalysatorabrieb an einem Staubabscheider oder in einem Elektrofilter als Reinigungszone abtrennt, wobei der Staubabscheider mit Schlauchfiltern ausgerüstet sein kann, die mit komprimiertem Kreislaufgas gereinigt werden. Es ist dann möglich, den in der Reinigungszone abgeschiedenen Katalysatorabrieb in einer nachgeschalteten Desorptionszone von adsorbierten Reaktionsprodukten zu befreien.

Bei einem solchen Verfahren wird es vermieden, daß bei der Ausschleusung des gebildeten Wassers sowie des bei der Aufarbeitung eingesetzten Waschwassers ein mit Schwermetall und anorganischem Schlamm verunreinigtes Abwasser entsteht. Nachteilig ist jedoch, daß der abgetrennte, nicht mehr zu verwendende Katalysatoranteil verworfen und sachgemäß entsorgt werden muß. Weiterhin ist bei einem derartigen Verfahren der Eintrag von PCDD/PCDF (polychlorierten Dibenzo-p-Dioxinen/Furanen) in die Umwelt über das Abwasser der Oxichlorierung immer noch erheblich. Auch hat sich dieses Verfahren als sehr aufwendig erwiesen und hat einen relativ hohen apparativen Aufwand und damit auch einen hohen Platzbedarf und hohe Investitionskosten zur Folge.

In der DE-A-197 53 165 wird ein Verfahren zur Herstellung von 1,2-Dichlorethan durch Oxichlorierung offenbart, wobei in einem Wirbelbett aus einem kupferhaltigen Katalysator Ethen mit Chlorwasserstoff und einem Sauerstoff enthaltenden Gas in Reaktion treten und das aus dem Reaktor austretende Reaktionsgas im Reaktor durch eine Feinstfiltration von Katalysator befreit und so im Reaktor zurückgehalten wird. Danach wird das vom Katalysator befreite Reaktionsgas in eine Quenchkolonne geleitet und in bekannter Art und Weise kondensiert.

Ein Nachteil dieses Verfahrens besteht in der Akkumulation der Feinstpartikel im Reaktor durch die Filtration. Dies ist verbunden mit einer Verschlechterung der Fließeigenschaften des Katalysatorbettes und einer Verschlechterung des Wärmeüberganges im Reaktor. Weiterhin ist unter anderem aufgrund der fehlenden Vorabscheidung durch die Reaktorzyklone die Staubbeladung des Reaktionsgases sehr hoch. Durch diesen hohen Partikelanteil muß die Filterfläche wesentlich vergrößert werden. Weiterhin lässt sich das in der DE 197 53 165 beschriebene Verfahren nur mit aufwendigen Mitteln auf bestehende Anlagen anwenden, so daß dieses Verfahren nur bei Neuanlagen zum Einsatz kommen wird und für bestehende Anlagen kaum in Frage kommt.

Weiterhin ist es aus dem Stand der Technik (Ullmann's Encyclopedia of Industria Chemistry, Vol. A6, 1986, S. 269) auch bekannt, die heißen Reaktionsgase aus dem Wirbelschichtreaktor der Oxychlorierung, die neben den Produkten 1,2-Dichlorethan und Wasser unter anderem auch nicht umgesetztes HCl-Gas enthalten, ohne weitere Behandlung nur mit einer wässrigen Lösung zu quenchen. Hierbei werden nicht abgeschiedener Katalysatorabrieb und nicht umgesetzter Chlorwasserstoff aus der Oxichlorierung von Ethen ausgewaschen. Als Waschflüssigkeit kann sowohl externes Wasser als auch Wasser, das bei der Reaktion entsteht, sogenanntes Reaktionswasser, verwendet werden. EDC- wird zusammen mit Wasser aus der Quenche abdestilliert und kondensiert.

Bei diesem Verfahren hat es sich als nachteilig erwiesen, daß das Abwasser aus der Quenche aufwendig entsorgt werden muß, da es unter anderem den Katalysatorabrieb enthält.

Alle bekannten Verfahren des Standes der Technik weisen den Nachteil auf, daß entweder nur eine ungenügende Abtrennung der PCDD/PCDF erfolgen kann oder aber eine ausreichende höhere Abtrennrate nur mit Hilfe aufwendiger Mittel oder durch komplizierte Verfahrensabläufe zu bewerkstelligen ist.

Es ist daher eine Aufgabe der vorliegenden Erfindung, ein Oxichlorierungsverfahren zur Herstellung von 1,2-Dichlorethan, unter Einsatz eines Wirbelbettes, derart durchzuführen, daß der bei der Reaktion entstehende Katalysatorabrieb mit möglichst wenig Aufwand entfernt werdenkann. Ferner soll dabei auch ein möglichst hoher Abscheidegrad des Katalysatorabriebes erreicht werden kann.

Es ist eine weitere Aufgabe der vorliegenden Erfindung, eine genügende Abtrennung der PCDD/PCDF von dem Reaktionsgas mit einem möglichst geringen apparativen Aufwand zu erreichen.

Diese Aufgaben werden bei einem Verfahren der eingangs genannten Art dadurch gelöst, daß das Reaktionsgas außerhalb des Oxichlorierungsreaktors mittels mindestens einer Filterkerze gefiltert wird.

Es wurde nämlich nun überraschenderweise gefunden, daß Filterkerzen weder hinsichtlich der leichten Durchführbarkeit der Abreinigung noch hinsichtlich der chemischen Beständigkeit (Korrosionsbeständigkeit) bei der Filterung des rohen 1,2-Dichlorethangasstromes unter üblichen Bedingungen Probleme bereiten. Diese Korrosionsbeständigkeit wurde deshalb nicht erwartet, weil die Filterkerzen eine extrem große Oberfläche aufweisen und bei Temperaturen von mindestens 200°C von korrosiven Gasgemischen durchströmt werden.

Gemäß einer bevorzugten Ausführungsform der vorliegenden Erfindung weist die mindestens eine Filterkerze als Filtermaterial mindestens ein keramisches Material und/oder mindestens ein gesintertes Metallpulver, und/oder mindestens ein Drahtvlies aus Edelstahl (1.4571 oder gleichwertige Materialien) oder einer anderen geeigneten Legierung auf.

Dabei können beispielsweise Legierungen, wie sie unter der Bezeichnung ®INCONEL; ®MONEL und/oder ®HASTELLOY handelsüblich sind, für die mindestens eine Filterkerze verwendet werden.

Ebenso können auch Filterkerzen, die keramische Materialien entsprechender mittlerer Porengrößen aufweisen, zum Einsatz kommen.

Bei Verwendung derartiger Filtermaterialien ist eine besonders gute Abscheidung von PCDD/PCDF aus dem Reaktionsgas möglich. Die Abscheideleistung bezogen auf Dioxine und Furane ist auch deshalb überraschend, da Stofftransporte dieser Verbindungen sehr komplex und weitgehend unerforscht sind. Insbesondere wenn die Filterkerzen, bzw. die bei den Filterkerzen verwendeten Filtermaterialien eine mittlere Porengröße oder eine Trenngrenze von ≤ 15 µm, bevorzugt von ≤ 8 µm, stärker bevorzugt von ≤ 5 µm oder von ≤ 1 µm aufweisen kann eine dramatische Verbesserung der Abscheidung von PCDD/PCDF erreicht werden. Als Untergrenze der mittleren Porengröße oder der Trenngrenze sind Werte von 0,0001 µm, 0,001 µm, 0,01 µm, 0,1 µm und 0,5 µm durchaus möglich. Somit können die Filtermaterialien z.B. mittlere Porengrößen von 0,0001 bis ≤ 5 µm aufweisen. Werden hingegen Gewebefilter eingesetzt, die bisher als gleichwirkend angesehen wurden, so kann keine Abtrennung von Dioxinen/Furanen erreicht werden.

Werden die Reaktionsgase zusätzlich einer direkten Kondensation d.h. einer Kondensation ohne Quenchung unterzogen, so gelangen die gegebenenfalls noch vorhandenen geringen Restmengen an PCDD bzw. PCDF nahezu quantitativ in die organische Phase und das Abwasser aus der Oxichlorierung ist weitestgehend frei von diesen Stoffen.

Da nun mit einfachsten Mitteln eine hohe Abscheideleistung insbesondere von polychlorierten Dibenzo-p-Dioxinen/Furanen (PCDD/PCDF) durch die Verwendung von mindestens eine Filterkerze aufweisenden, außenliegenden Filterelementen erreicht werden kann, ist eine drastische Reduzierung des Gehaltes an derartigen Verbindungen im Abwasser möglich, das ansonsten aufwendig und teuer aufgearbeitet werden muß.

Das Problem des Absetzens der Feinstpartikel im und damit des Verstopfens der Filtergehäuse konnte durch folgende Strömungsführung des Gases im Filter gelöst werden: Der in Richtung der Schwerkraft gerichtete resultierende Geschwindigkeitsvektor aus der Sinkgeschwindigkeit des Katalysatorteilchen und der Strömungsgeschwindigkeit des Gases (Kreislaufgas, 1,2 Dichlorethan und Prozeßwasser) ist größer 0.

Wie bereits beschrieben, ist es bei bestimmten Anwendungen sogar denkbar, daß das Reaktionsgas nach einer Feinstfiltration ohne vorherige Quenchung direkt kondensiert wird, so daß der bisher im Stand der Technik immer nötwendige Verfahrensschritt der Quenchung entfallen kann. Dadurch wird das Oxichlorierungsverfahren noch einfacher und kostengünstiger. Bei einem derartigen Verfahren kann somit eine Vorrichtung verwendet werden, bei der kein sogenannter Quenchturm notwendig ist, was eine Platzersparnis bei der zu verwendenden Vorrichtung zur Folge hat.

Die Verfahrensbedingungen, insbesondere die des Oxichlorierungsschrittes mittels Wirbelbett, können vorzugsweise in Übereinstimmung mit den in der deutschen Auslegeschrift 1 518 931 und dem deutschen Patent 1 468 489 beschriebenen Verfahrensbedingungen durchgeführt werden, deren Offenbarung hiermit durch Bezugnahme in die vorliegende Beschreibung aufgenommen wird.

Vorzugsweise durchströmt das Reaktionsgas nach Verlassen des Oxichlorierungsreaktors bei einem erfindungsgemäßen Verfahren die Filterkerze mit einem Druck von etwa 1 bis 6 bar, vorzugsweise von etwa 3,5 bar.

Dabei sollte das Reaktionsgas beim Durchströmen des Filters eine Temperatur von etwa 200 bis 250°C vorzugsweise etwa 220°C aufweisen.

Besonders gute Ergebnisse konnten bei einem erfindungsgemäßen Verfahren erzielt werden, wenn der in Richtung der Schwerkraft gerichtete resultierende Geschwindigkeitsvektor aus der Sinkgeschwindigkeit des Katalysatorteilchen und der Strömungsgeschwindigkeit des Gases (Kreislaufgas, 1,2 Dichlorethan und Prozeßwasser) größer 0, inbesondere größer 10 mm /Sekunde ist.

Nach dem Filtern könnte je nach Reinigungsgrad das Reaktionsgas in eine Quenchkolonne geleitet oder direkt kondensiert werden.

Bei einem derartigen Verfahren ist es auch möglich, eine sehr einfach aufgebaute Vorrichtung zu verwenden, da das Entfernen des Katalysatorabriebes nicht mittels anderer aufwendiger Apparaturen, wie sie beispielsweise in der DE 197 53 165 beschrieben werden, erfolgen muß.

Vorzugsweise beträgt der Anteil an polychlorierten Dibenzo-p-Dioxinen/Furanen (PCDD/PCDF) im 1,2-Dichlorethan nach einem erfindungsgemäßen Verfahren weniger als 0,1 µg/to 1.2 Dichlorethan.

Der Abscheidegrad der mitgerissenen Katalysatorteilchen beim Oxichlorierungsverfahren mittels Wirbelbett beträgt bei einem erfindungsgemäßen Verfahren gemäß einer bevorzugten Ausführungsform mehr als 99,99 %.

Dadurch, daß die Filtration mit einer zur Durchführung eines erfindungsgemäßen Verfahren verwendeten außerhalb des Oxichlorierungsreaktors befindlichen Filterkerze erfolgt, ist in einfachster Weise ein Nachrüsten bestehender Oxichlorierungsanlagen mit Wirbelbett möglich. Dies kann insbesondere auch deshalb sehr einfach erfolgen, da die verwendeten Filter mit mindestens einer Filterkerze sehr kompakt gebaut werden können.

Aufgrund der hohen Betriebssicherheit der Filterkerzen ist es darüber hinaus auch möglich, auf eine Reserveeinheit zu verzichten.

Unter anderem deshalb, weil die Filterkerzen praktisch keinem Verschleiß unterliegen, haben diese eine unbegrenzte Lebensdauer zumal bei der erfindungsgemäßen Stromführung in der Filterkerze eine leichte Entfernung der abfiltrierten Teilchen möglich ist. Auch dies hat sich als Vorteil erwiesen, da keine aufwendigen Wartungsarbeiten durchgeführt werden müssen.

Insbesondere um ein erfindungsgemäßes Verfahren besonders gut durchführen zu können, sollte die Vorrichtung zur Herstellung von 1,2-Dichlorethan nach dem Oxichlorierungsverfahren in einem Wirbelschichtreaktor Zuführungen für Chlorwasserstoff und Sauerstoff aufweisendes Gas aufweisen, die direkt in das Wirbelbett des Oxichlorierungsreaktors führen.

Diese Zuführungen können dabei aus porösen, gasdurchlässigen Formkörpern gebildet sein.

Auch ist es besonders günstig, wenn das Ethen und der Kreisgasstrom über einen Boden, der aus porösem, gasdurchlässigen Material gefertigt ist oder mit Formkörpern aus porösem, gasdurchlässigen Material versehen ist, in den Oxichlorierungsreaktor erfolgt, welches die Ausbildung des Wirbelbettes unterstützt.

Die Ausgestaltung der Zuleitungen könnte beispielsweise in der in der DE 199 03 335 A1 beschriebene Art und Weise gewählt sein, die hier unter Bezugnahme mit in die Beschreibung aufgenommen werden soll.

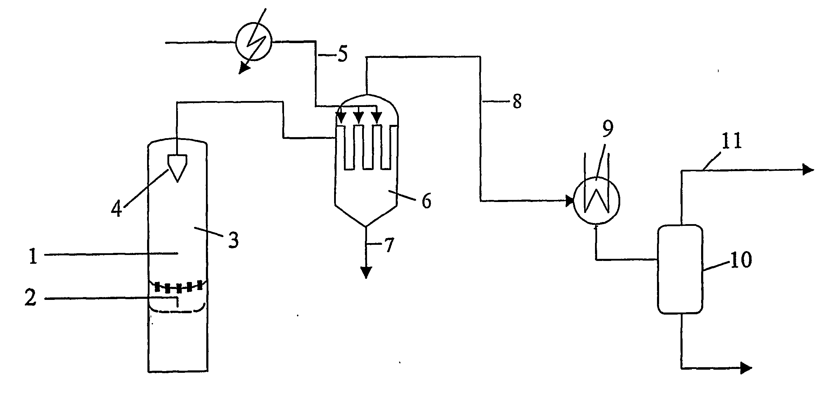

Weitere Vorteile und Weiterbildungen der Erfindung ergeben sich aus den Patentansprüchen, der Zeichnung und der nachfolgenden Beschreibung, in der unter Bezugnahme auf die Zeichnung Ausführungsbeispiele der Erfindung im einzelnen beschrieben sind.

Es zeigt dabei die einzige Figur beispielhaft eine erfindungsgemäße Vorrichtung gemäß einer bevorzugten. Ausführungsform.

Auch das Verfahren nach der Erfindung wird nachstehend anhand der Zeichnung erläutert.

Es werden über eine Rohrleitung 1 die vorgewärmten Edukte Chlorwasserstoff und Sauerstoff direkt in einen Reaktionsraum eines Reaktors 3 geleitet. Gleichzeitig werden über eine weitere Rohrleitung 2 das vorgewärmte Kreislaufgas und Ethen in den Reaktor 3 geführt. Im Reaktor 3 befindet sich ein Katalysator, der in der Figur nicht dargestellt ist. Der rohe 1,2 Dichlorethanstrom, das Prozeßwasser und das Kreislaufgas werden über Zyklone 4 in den Feinstfilter 6 geleitet. Der abgetrennte Katalysator-Feinststaub wird über eine Schleuse 7 aus dem System ausgeschleust. Über eine dritte Rohrleitung 5 wird das vorgeheizte Rückspülgas zum Abreinigen (Rückspülen) der Filterelemente in den Filter 6 geleitet. Der rohe 1,2 Dichlorethanstrom, das Prozeßwasser und das Kreislaufgas ohne signifikanten Feinstpartikel Anteil werden über eine weitere Rohrleitung 8 in einen Kondensator 9 geleitet, in dem der EDC - und der Wasserdampf kondensiert werden. Das flüssige EDC / Wassergemisch wird in einem Abscheider 10 vom Kreisgas getrennt und einer Aufarbeitung über eine Rohrleitung 12 zugeführt. Das Kreisgas wird über eine Rohrleitung 11 wieder auf die Saugseite des Kreisgasverdichters zurückgeführt.

### Beispiel 1

Es wird zur Herstellung von 1,2-Dichlorethan ein Oxichlorierungsreaktor mit einem Wirbelbett verwendet, wobei als Katalysator CuCl₂ verwendet wird. Die Oxichlorierung wird dabei unter üblichen Verfahrensbedingungen durchgeführt, die dem Fachmann bekannt sind und deshalb nicht explizit genannt werden.

Das Reaktionsgas (500 Nm³/h) durchströmt nach Verlassen des Wirbelschichtreaktors einen Feinstfilter einer trocken betriebenen Reinigungszone bei einer Temperatur von 200 bis 250°C (vorzugsweise 220°C) und einem Druck von etwa 1 bis 6 bar (vorzugsweise 3,5 bar) zur Abscheidung von mitgerissenen Teilchen des Katalysators, dem sogenannten Katalysatorabrieb, wodurch der Katalysator praktisch vollständig abgeschieden wird.

Der Feinstfilter besteht aus 8 Filterelementen. Die Filterelemente sind aus dem Werkstoff 316 S (stainless steel) gefertigt. Die 8 Filterelemente werden über automatische Ventile periodisch rückgespült. Als Rückspülgas dient vorgewärmter Stickstoff, der mit einem Druck von 8 bar zur Verfügung steht. Der im Filterkonus abgeschiedene Feinststaub wird 1x/Tag über eine Schleuse entfernt.

Die Führung des Reaktionsgases ist dabei derart vorgesehen, daß der in Richtung der Schwerkraft gerichtete resultierende Geschwindigkeitsvektor aus der Sinkgeschwindigkeit des Katalysatorteilchen und der Strömungsgeschwindigkeit des Gases (Kreislaufgas , 1,2 Dichlorethan und Prozeßwasser) größer 0 ist.

Dadurch wird ein optimales Absetzen der Feinstpartikel gewährleistet.

Das vom Katalysatorabrieb befreite Reaktionsgas mit einer Temperatur von 200 bis 250°C, vorzugsweise etwa 220°C wird dann über eine Leitung in einen Kondensator geleitet, wo das EDC und das Produktionswasser kondensiert werden. In einem Gasabscheider wird die kondensierte Flüssigkeit vom Kreisgas getrennt. Das EDC / Wassergemisch wird über eine Rohrleitung einem Trennbehälter zugeführt, in dem die Wasserphase vom EDC getrennt wird.

Folgende Konzentrationen an polychlorierten Dibenzo-p-Dioxinen/Furanen wurden im Abwasser der Oxychlorierung gemessen:

| | Ohne Filter | Gewebefilter | Sintermetallfilter (rost-freier Stahl) mit einer mittleren Porengröße < 5 µm |
|---|---|---|---|
| PCDD/PCDF TEQ nach NATO/CCMS in µg/t OC Kapazität | 5 | 4,5 | <0,1 |

| | | | |
|---|---|---|---|
| Erklärung der Abkürzungen: PCDD = Polychlorierte Dibenzodioxine PCDF = Polychlorierte Dibenzofurane TEQ= Toxizitäts Equivalent NATO/CCMS = North Atlantic Treaty Organization/Committee on the Challenges of Modern Society µg = Mikrogramm t OC = Tonne Oxychlorierungskapazität | | | |

Das bedeutet, daß es mit einem erfindungsgemäßen Verfahren möglich wird, den PCDD/PCDF-Gehalt nach der Filtration mittels Filterkerzen einer speziellen mittleren Porengröße gegenüber der Filtration mit überlicherweise im Stand der Technik verwendeten Gewebefiltern um mehr als 97 % zu verringern.

Der Abscheidegrad von mitgerissenen Katalysatorteilchen liegt somit bei mehr als 99,99 %. Damit kann in Folge dessen der Gehalt an polychlorierten Dibenzo-p-Dioxinen/Furanen im Abwasser drastisch reduziert werden. Es muß daher nicht mehr eine sehr kostenintensive und zeitintensive Entsorgung des Abwassers nach der Oxichlorierung erfolgen.

## Patentansprüche

1. Verfahren zur Herstellung von 1,2-Dichlorethan durch Umsetzen von Ethen mit Chlorwasserstoff und einem Sauerstoff aufweisenden Gas in einem Oxichlorierungsreaktor unter Bildung eines Reaktionsgases,
**dadurch gekennzeichnet,**
**daß** das Reaktionsgas außerhalb des Oxichlorierungsreaktors mittels mindestens einer Filterkerze gefiltert wird.

2. Verfahren nach Anspruch 1,
**dadurch gekennzeichnet,**
**daß** das für die mindestens eine Filterkerze verwendete Filtermaterial keramische Materialien, gesinterte Metallpulver, Drahtvlies aus Edelstahl oder Edelstahllegierungen umfaßt.

3. Verfahren nach einem der vorstehenden Ansprüche, **dadurch gekennzeichnet,**
**daß** das für die mindestens eine Filterkerze verwendete Filtermaterial eine mittlere Porengröße von ≤ 15 µm, vorzugsweise von ≤ 8 µm und stärker bevorzugt von ≤ 5 µm aufweist.

4. Verfahren nach einem der vorstehenden Ansprüche,
**dadurch gekennzeichnet,**
**daß** das Reaktionsgas die Filterkerze mit einem Druck von etwa 1 bis 6 bar durchströmt.

5. Verfahren nach einem der vorstehenden Ansprüche,
**dadurch gekennzeichnet,**
**daß** das Reaktionsgas die Filterkerze mit einer Temperatur von etwa 200 bis 250°C durchströmt.

6. Verfahren nach einem der vorstehenden Ansprüche,
**dadurch gekennzeichnet,**
**daß** die Strömungsführung des Gases in der Filterkerze derart gewählt ist, daß der in Richtung der Schwerkraft gerichtete resultierende Geschwindigkeitsvektor aus der Sinkgeschwindigkeit der zu filtrierenden Teilchen wie den Katalysatorteilchen und der Strömungsgeschwindigkeit des Gases (Kreislaufgas, 1,2 Dichlorethan und Prozeßwasser) größer 0 ist.

7. Verfahren nach einem der vorhergehenden Ansprüche,
**dadurch gekennzeichnet,**
**daß** nach dem Filtern das Reaktionsgas in eine Quenchkolonne geleitet oder direkt kondensiert wird.

8. Verfahren nach einem der vorhergehenden Ansprüche,
**dadurch gekennzeichnet,**
**daß** der Anteil polychlorierter Dibenzo-p-Dioxine/Furane (PCDD/PCDF) weniger als 0,1 µg/t 1,2 Dichlorethan im Abwasser der Wirbelbett Oxychlorierung beträgt.

9. Verfahren nach einem der vorhergehenden Ansprüche,
**dadurch gekennzeichnet,**
**daß** der Abscheidegrad von Katalysatorabrieb aus dem Reaktionsgas mehr als 99,99 % beträgt.

10. Vorrichtung zur Herstellung von 1,2-Dichlorethan durch Umsetzen von Ethen mit Chlorwasserstoff und einem Sauerstoff aufweisenden Gas, insbesondere zur Verwendung bei einem Verfahren nach einem der vorhergehenden Ansprüche, die einen Oxichlorierungsreaktor und einen Filter aufweist,
**dadurch gekennzeichnet,**
**daß** der Filter mindestens eine Filterkerze aufweist, die außerhalb des Oxichlorierungsreaktors angeordnet ist und das Reaktionsgas filtriert.

11. Vorrichtung nach Anspruch 10,
**dadurch gekennzeichnet,**
**daß** die mindestens eine Filterkerze keramische Materialien, gesinterte Metallpulver, Drahtvliese aus Edelstahl oder Edelstahllegierungen aufweist.

12. Vorrichtung nach Anspruch 10 oder 11,
**dadurch gekennzeichnet, daß** das für die mindestens eine Filterkerze verwendete Filtermaterial ®INCONEL, ®MONEL und/oder ®HASTELLOY umfaßt.

13. Vorrichtung nach einem der Ansprüche 10 - 12,
**dadurch gekennzeichnet,**
**daß** das für die mindestens eine Filterkerze verwendeten Filtermaterialien eine mittlere Porengröße von 0,01 - 15 µm, vorzugsweise von 0,1 - 8 µm stärker bevorzugt von 0,2 bis 5 µm und am stärksten bevorzugt von 0,8 bis 3 µm aufweist.

14. Vorrichtung nach einem der Ansprüche 10 bis 13 **dadurch gekennzeichnet,**
**daß** die Zuführungen für Chlorwasserstoff und/oder das Sauerstoff aufweisende Gas direkt in das Wirbelbett eines Wirbelschichtreaktors geführt sind.

15. Vorrichtung nach Anspruch 14,
**dadurch gekennzeichnet,**
**daß** die Zuführungen poröse, gasdurchlässige Formkörpern aufweisen.

16. Vorrichtung nach einem der Ansprüche 10 bis 15
**dadurch gekennzeichnet,**
**daß** das Kreisgas und/oder das Ethen durch einen poröses, gasdurchlässiges Material aufweisenden Boden in den Oxichlorierungsreaktor geführt ist.

## Claims

1. Process for the preparation of 1,2-dichloroethane by reacting ethene with hydrogen chloride and an oxygen-containing gas in an oxychlorination reactor with formation of a reaction gas,
**characterised in that**
the reaction gas is filtered outside the oxychlorination reactor by means of at least one filter candle.

2. Process according to claim 1,
**characterised in that**
the filter material used for the at least one filter candle comprises ceramic materials, sintered metal powders, wire fleece of high-grade steel or high-grade steel alloys.

3. Process according to one of the preceding claims, **characterised in that**
the filter material used for the at least one filter candle has an average pore size of ≤ 15 µm, preferably ≤ 8 µm and more preferably ≤ 5 µm.

4. Process according to one of the preceding claims,
**characterised in that**
the reaction gas flows through the filter candle at a pressure of about from 1 to 6 bar.

5. Process according to one of the preceding claims,
**characterised in that**
the reaction gas flows through the filter candle at a temperature of about from 200 to 250°C.

6. Process according to one of the preceding claims,
**characterised in that**
the routing of the gas stream in the filter candle is so selected that the speed vector directed in the direction of gravity, resulting from the sinking speed of the particles being filtered out, such as the catalyst particles, and the flow speed of the gas (recycle gas, 1,2-dichloroethane and process water), is greater than 0.

7. Process according to one of the preceding claims,
**characterised in that**,
after filtering, the reaction gas is passed into a quenching column or is directly condensed.

8. Process according to one of the preceding claims,
**characterised in that**
the content of polychlorinated dibenzo-p-dioxins/furans (PCDD/PCDF) is less than 0.1 µg/t of 1,2-dichloroethane in the waste water from fluidised-bed oxychlorination.

9. Process according to one of the preceding claims,
**characterised in that**
the degree of separation of catalyst fragments from the reaction gas is more than 99.99 %.

10. Apparatus for the preparation of 1,2-dichloroethane by reacting ethene with hydrogen chloride and an oxygen-containing gas, especially for use in a process according to one of the preceding claims, which has an oxychlorination reactor and a filter,
**characterised in that**
the filter has at least one filter candle, which is arranged outside the oxychlorination reactor and filtering the reaction gas.

11. Apparatus according to claim 10,
**characterised in that**
the at least one filter candle comprises ceramic materials, sintered metal powders, wire fleeces of high-grade steel or high-grade steel alloys.

12. Apparatus according to claim 10 or 11,
**characterised in that**
the filter material used for the at least one filter candle comprises ®INCONEL, ®MONEL and/or ®HASTELLOY.

13. Apparatus according to one of claims 10 - 12,
**characterised in that**
the filter material used for the at least one filter candle has an average pore size of from 0.01 to 15 µm, preferably from 0.1 to 8 µm, more preferably from 0.2 to 5 µm, and most preferably from 0.8 to 3 µm.

14. Apparatus according to one of claims 10 to 13,
**characterised in that**
the inlets for hydrogen chloride and/or the oxygen-containing gas pass directly into the fluidised bed of a fluidised-bed reactor.

15. Apparatus according to claim 14,
**characterised in that**
the inlets comprise porous, gas-permeable packing elements.

16. Apparatus according to one of claims 10 to 15,
**characterised in that**
the recycle gas and/or the ethene is passed into the oxychlorination reactor through a tray of porous, gas-permeable material.

## Revendications

1. Procédé de préparation de 1,2-dichloroéthane par réaction d'éthylène avec du chlorure d'hydrogène et avec un gaz contenant de l'oxygène, dans un réacteur d'oxychloration aboutissant à la formation d'un gaz réactionnel, **caractérisé par le fait que** le gaz réactionnel est filtré à l'extérieur du réacteur d'oxychloration au moyen d'au moins une bougie filtrante.

2. Procédé selon la revendication 1, **caractérisé par le fait que** le matériau filtrant utilisé pour la ou les bougies filtrantes est un matériau céramique, une poudre métallique frittée, un non-tissé de fils métalliques en acier inoxydable ou en alliage d'acide inoxydable.

3. Procédé selon l'une des revendications précédentes, **caractérisé par le fait que** le matériau filtrant utilisé pour la ou les bougies filtrantes a une taille moyenne de pores de ≤ 15 µm, de préférence de ≤ 8 µm, et en particulier de ≤ 5µm.

4. Procédé selon l'une des revendications précédentes, **caractérisé par le fait que** le gaz réactionnel traverse la bougie filtrante à une pression d'environ 1 à 6 bars.

5. Procédé selon l'une des revendications précédentes, **caractérisé par le fait que** le gaz réactionnel traverse la bougie filtrante à une température de environ 200 à 250 °C.

6. Procédé selon l'une des revendications précédentes, **caractérisé par le fait que** l'écoulement du flux gazeux dans la bougie filtrante est tel que le vecteur vitesse résultant du vecteur vitesse de sédimentation des particules à filtrer comme les particules de catalyseur et du vecteur vitesse d'écoulement des gaz (gaz de recyclage, 1,2-dichloroéthane et eau de procédé) est supérieur à 0 et dirigé dans le sens de la force de gravité.

7. Procédé selon l'une des revendications précédentes, **caractérisé par le fait que** le gaz réactionnel est amené, après l'étape de filtration, dans une colonne de refroidissement ou est soumis directement à une condensation.

8. Procédé selon l'une des revendications précédentes, **caractérisé par le fait que** la concentration de dibenzo-p-dioxines/furannes (PCDD/PCDF) dans les eaux résiduaires provenant de l'oxychloration en lit fluidisé est inférieure à 0,1 µg/tonne de 1,2-dichloroéthane.

9. Procédé selon l'une des revendications précédentes, **caractérisé par le fait que** le taux de séparation des fines de catalyseur à partir du gaz réactionnel est supérieur à 99,99 %.

10. Installation pour la préparation de 1,2-dichloroéthane par réaction d'éthylène avec du chlorure d'hydrogène et avec un gaz contenant de l'oxygène, en particulier destinée à être utilisée dans un procédé selon l'une quelconque des revendications précédentes, comportant un réacteur d'oxychloration et un filtre, **caractérisé par le fait que** le filtre comprend au moins une bougie filtrante disposée à l'extérieur du réacteur d'oxychloration et filtrant le gaz réactionnel.

11. Installation selon la revendication 10, **caractérisée par le fait que** le matériau utilisé pour la ou les bougies filtrantes est un matériau céramique, une poudre métallique frittée, un non-tissé de fils métalliques en acier inoxydable ou en alliage d'acide inoxydable.

12. Installation selon la revendication 10 ou 11, **caractérisée par le fait que** le matériau filtrant utilisé pour la ou les bougies filtrantes englobe les produits INCONEL^{®}, MONEL^{®} et/ou HASTELLOY^{®}.

13. Installation selon l'une des revendications 10 à 12, **caractérisée par le fait que** les matériaux filtrants constituant la ou les bougies filtrantes ont une taille moyenne de pores de 0,01 à 15 µm, de préférence de 0,1 à 8 µm, en particulier de 0,2 à 5 µm et idéalement de 0,8 à 3 µm.

14. Installation selon l'une des revendications 10 à 13, **caractérisée par le fait que** les arrivées de chlorure d'hydrogène et/ou de gaz contenant de l'oxygène aboutissent directement dans le lit fluidisé d'un réacteur à lit fluidisé.

15. Installation selon la revendication 14, **caractérisée par le fait que** les arrivées de gaz comportent des éléments moulés poreux, perméables aux gaz.

16. Installation selon l'une des revendications 10 à 15, **caractérisé par le fait que** le gaz de recyclage et/ou l'éthylène arrivent dans le réacteur d'oxychloration par le fond de celui-ci qui est en un matériau poreux, perméable aux gaz.
